# EUROPEAN PATENT SPECIFICATION

(11) **EP 0 783 266 B1**
(45) Date of publication and mention of the grant of the patent: **02.05.2003**
(21) Application number: 94929235.3
(22) Date of filing: 16.09.1994
(51) Int. Cl.: A61B 5/055, A61B 6/04, G01R 33/34, G01R 33/28

(54) **MAGNETIC RESONANCE IMAGING DEVICE FOR TOOL GUIDING**
MAGNETISCHE RESONANZDARSTELLUNGSVORRICHTUNG ZUR WERKZEUGFÜHRUNG
APPAREIL D'IMAGERIE PAR RESONANCE MAGNETIQUE POUR LE GUIDAGE D'UN INSTRUMENT

(43) Date of publication of application: 16.07.1997
(73) Proprietor: FISCHER IMAGING CORPORATION, Denver, CO 80241 (US)
(72) Inventor: SICZEK, Bernard, Boulder, CO 80303 (US)
(74) Representative: Bockhorni, Josef, Dipl.-Ing.
(86) International application number: US9410533
(87) International publication number: WO96008199

(56) References cited:
- WO-A-93/17620
- WO-A-94/06352
- DE-A- 4 325 206
- US-A- 4 608 991

## Description

### FIELD OF THE INVENTION

This invention relates generally to breast imaging and, in particular, to a device which employs magnetic resonance imaging for locating an area of interest within a patient's breast and which is also useful for guiding a medical instrument to the area of interest. The device is particularly useful for sampling, excising or treating breast lesions including lesions suspected of being cancerous.

### BACKGROUND OF THE INVENTION

X-ray mammography has long been considered the "gold standard" of breast imaging and is well-developed and capable of yielding outstanding image resolution. In x-ray mammography, x-rays are transmitted through the patient's breast and impinge upon x-ray film or a digital imaging camera. Internal features of the patient's breast are depicted as shadows in the resulting image due to differences in x-ray absorption between differing types of tissue. Ordinarily, the patient's breast is compressed during x-ray mammography so that the breast is of more uniform thickness, thereby enhancing the resulting image.

In addition to pure imaging applications such as patient screening or diagnosis, x-ray mammography has been used to localize breast lesions for needle biopsy or for placement of a wire or markings to guide a surgeon during subsequent surgical biopsy.

Recently, magnetic resonance imaging (MRI) has been investigated for use in breast imaging. Generally, in MRI, atomic nuclei which possess nuclear magnetic momentum, or spin, are first aligned in a relatively strong magnetic field and are then excited by radio frequency (RF) resonance energy. When their spin returns to equilibrium they emit energy in the form of an RF signal. The time of emission of the absorbed energy, called the relaxation time, depends on magnetic properties of the tissue. The emitted signal can be analyzed to obtain imaging information regarding the tissue under examination.

Ordinarily, specified regions or slices of the tissue are sequentially examined so that a composite image of the tissue under examination is obtained.

MRI is of interest to breast imaging specialists for various reasons. Initially, MRI avoids cumulative radiation exposure, a concern expressed by some in connection with alternative x-ray imaging techniques.

Additionally, MRI reduces the need to compress the patient's breast to a uniform thickness, thereby potentially enhancing patient comfort. Moreover, MRI may provide imaging advantages for certain soft tissue imaging applications due to its reliance on nuclear magnetic relaxation times rather than x-ray absorption or other characteristics. Advances in MRI technology are also improving MRI resolution.

However, as to breast imaging, a number of challenges remain in realizing the full potential of MRI. For example, conventional tunnel MRI devices where a patient is axially positioned within a magnetic tunnel are intended for obtaining a composite image from axial body slices. These systems are not tailored for breast imaging and, standing alone, are not capable of yielding the images necessary for increasingly acute breast diagnostic techniques.

Moreover, known MRI devices are not adapted for use in localizing a breast lesion so as to permit insertion of a medical instrument to sample, excise or treat the lesion.

DE 43 252 06 A1 discloses a stereotactic auxiliary attachment for a nuclear magnetic resonance tomography apparatus for use in conducting mammographic examinations. The auxiliary attachment includes two parallel compression plates to hold a patient's breast in a fixed position and at least one reception coil of the magnetic resonance tomography apparatus defining volume dimensions for receiving the patients breast.

WO 93/17620 discloses an isocentric puncture instrument aiming device for targeting an area of interest within a patient's breast. The system uses compression paddles to compress and to flatten the patients breast, similar to the plates used in DE 43 252 06 A1, for improved X-ray imaging.

With regard to conventional tunnel MRI devices, due to space limitations within the tunnel bore, it is impractical to perform procedures involving insertion of a medical instrument into a patient's breast while the patient remains in an imaging position within the bore. In addition, such devices do not provide a mechanism for allowing localization of a breast lesion after the patient has been withdrawn from the tunnel bore. Known MRI devices which are dedicated to breast imaging generally include magnetic generators and signal transmitters/receivers which surround the patient's breast. These components can interfere with conventional medical instrument insertion devices.

### SUMMARY OF THE INVENTION

The present invention provides an apparatus such as an MRI device for use in perfoming medical procedures on a patient's breast as defined in claim 1. Favorable embodiments are listed in dependent claims 2 - 12.

Such an MRI device is generally useful for imaging a patient's breast, and particularly apt for localizing an area of interest within the patient's breast and readily and accurately obtaining a sample therefrom by insertion of a medical instrument. The invention can advantageously utilize elements of a conventional tunnel MRI device, as is often available on premises, thereby simplifying device construction.

Additionally, the device provides for breast specific MRI without interfering with medical device insertion.

According to the invention, an MRI/guidance device includes a breast imaging assembly comprising an MRI signal receiver, such as a coil for receiving radio frequency signals, which is dimensioned to receive the breast under examination. The receiver coil can also be utilized, in cooperation with a magnetic field generator for producing a local magnetic field, to transmit an MRI signal.

The transmitted MRI signal, or just the magnetic field employed in transmitting the signal, can be provided by a conventional tunnel MRI device. A specialized table may be provided for supporting the patient and a signal receiver and for permitting the patient and receiver to be introduced into the bore of a conventional tunnel MRI device. The table supports the patient in a prone position and includes an opening through which the patient's breast is permitted to pendulantly protrude. The receiver is supported by the table such that the receiver surrounds the patient's pendulant breast. In this manner, elements of a conventional tunnel NRI device can be utilized while obtaining the advantages of a localized receiver. The table is moveable between an extended position where the patient's breast is centrally disposed within the bore of the MRI tunnel and a retracted position where the patient is withdrawn from the bore. A mechanism such as bumpers, detents or the like is utilized to ensure accurate registration of the table in the extended and retracted positions.

The the MRI/guidance device also includes an assembly for immobilizing the patient's breast such that information obtained via MRI can subsequently be used for identifying an area of interest within the patient's breast and for inserting a medical instrument to the identified area of interest. The patient's breast is immobilized by a contractible basket which engages the patient's breast. The basket is disposed between the signal receiver and the patient's breast.

Preferably, the basket is shaped to generally conform to the contours of the patient's breast and includes a number of openings to allow for insertion of a medical instrument therethrough. In one embodiment, the basket is formed in two halves which are interconnected using a spring or other resilient member so that the halves are drawn towards a contracted position to engage the patient's breast.

Further, the MRI/guidance device includes a medical instrument guidance assembly for aiming a medical instrument to an identified area of interest within the patient's breast. Based on NRI information, the guidance assembly directs a medical instrument towards the area of interest via a penetration path which avoids obstructions such as the signal receiver and/or breast immobilization assembly.

In this regard, the guidance assembly supports the medical instrument so that the medical instrument is moveable between at least a first position and a second position wherein the medical instrument is aimed at a common point, the isocenter, in each of the first and second positions. For example, the medical instrument can be orbitally moved relative to the isocenter so that the medical instrument pivots thereabout in a single plane (defined by movement of the aiming axis) and/or the medical instrument can be rotationally moved relative to the isocenter so that the puncture instrument pivots thereabout in three-dimensions. Aiming the medical device at the area of interest is accomplished by moving the patient and/or guidance assembly so that the area of interest coincides with the isocenter. The medical instrument can then be inserted to the area of interest for sampling, excision or treatment as desired.

The present invention thus allows for use of a conventional tunnel MRI device while obtaining the advantages of localized, breast specific signal reception.

The invention also allows for insertion of a medical instrument to an identified area of interest within the patient's breast, e.g., a breast lesion, under MRI guidance. Further advantages of the present invention will be appreciated upon consideration of the Detailed Description below taken in conjunction with the Drawings.

### BRIEF DESCRIPTION OF THE DRAWINGS

Fig. 1 is a side view of a MRI/guidance device constructed in accordance with the present invention;
Fig. 2a is a side view of the medical instrument guidance assembly which forms a portion of the device of Fig. 1;
Fig. 2b is a cross-sectional view of the medical instrument guidance assembly of Fig. 2a illustrating the slidable interconnection which facilitates orbital movement of the medical instrument;
Fig. 3a is a bottom view of the device of Fig. 1 illustrating a technique for positioning the breast immobilization basket and signal receiver coil relative to the patient;
Fig. 3b is a bottom view showing the breast immobilization basket assembly; and
Fig. 3c is a front view of the breast immobilization basket.

### DETAILED DESCRIPTION

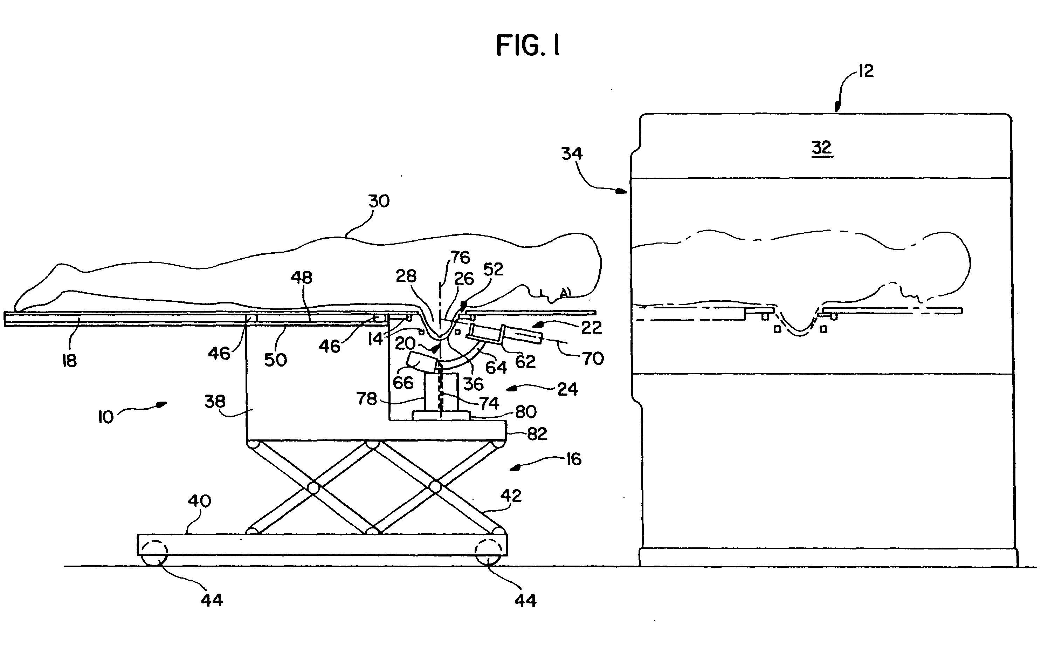

Referring to Figs. 1-3c, an MRI/guidance system is generally identified by the reference numeral 10. The system 10 comprises a magnetic field generator 12, a signal receiver 14, a stretcher assembly 16 including an extendible table top 18, a breast immobilization basket assembly 20, a medical instrument insertion assembly 22, and a guidance assembly 24 for aiming a medical instrument 26.

The system 10 employs known MRI principles to image a breast 28 of a patient 30. As is known, MRI generally involves subjecting the tissue to be imaged to a strong magnetic field, applying an RF signal to the tissue and receiving an RF signal from the tissue. In accordance with the present invention, the magnetic field may be specifically directed to the breast under examination via a dedicated magnetic field generator or the less localized magnetic field of a tunnel MRI device may be utilized.

In the illustrated embodiment, the magnetic field generator 12 comprises a conventional tunnel MRI device as is commonly available at medical imaging facilities, thereby eliminating the need for a magnetic field generator dedicated to breast imaging applications. The generator 12 comprises a resistive or superconductive magnet 32 which is generally configured in the shape of a cylinder. The illustrated generator can further include a shim coil to correct magnet field inhomogeneities and a gradient coil for producing a magnetic field gradient for encoding spatial information into the imaging signals. In addition, conventional tunnel MRI systems normally include a body coil for applying an RF signal to the patient 30. As will be described below, this RF signal can alternatively be applied in accordance with the present invention via receiver 14. These components are disposed about a central bore 34 of sufficient size to receive the patient 30 therein. The generator 12 may comprise, for example, the SIGMA system manufactured by General Electric.

Stretcher assembly 16 is utilized for transporting the patient 30 into and out of bore 34. The stretcher assembly 16 comprises an extendible table top 18 supported by platform 38. Platform 38, in turn, is supported on base 40 via collapsible platform jack 42 so as to allow for adjustment of the height of table top 18. In this regard, the height of table top 18 is preferably adjusted so that the breast 28 under examination is centrally disposed within bore 34 for optimal imaging. Conveniently, assembly 16 can be provided with rollers to facilitate positioning of assembly 16 relative to generator 12.

Table top 18 is axially moveable relative to bore 34 between an extended position wherein the patient 30 is disposed within bore 34 for imaging (shown in phantom in Fig. 1) and a retracted position for performing further medical procedures on the patient's breast 28. To facilitate movement of table top 18, rollers 46 are provided on upper surface 48 of platform 38. The rollers 46 roll in tracks formed in lower surface 50 of table top 18 to ensure that movement of table top 18 is aligned axially with bore 34. The rollers 46 and the tracks of table top 18 should be formed from non-magnetic materials such as stainless steel to provide for smooth rolling movement of table top 18. It will be appreciated that, in the illustrated system 10, all components utilized in close proximity to magnetic generator 12 are formed from materials which do not affect the homogeneity of the magnetic field used. To obtain good images, the magnetic field has to be homogeneous to a very high degree.

As will be understood upon consideration of the description below, it is useful to provide for positive registration of the table top 18 in the extended and retracted positions. In this regard, bumpers can be provided on the upper surface 48 of platform 38 and/or lower surface 50 of table top 18 to positively limit table top movement. Alternatively, a detent may be provided on one of these surfaces or elsewhere to accurately control the relative positioning of table top 18 and platform 38.

As shown, the patient 30 lies in a prone position on table top 18. At least one opening 52 is provided in table top 18 to allow the breast 28 under examination to pendulantly protrude through the table top 18. The illustrated table top includes two openings 52 positioned so that either of the patient's breasts can be examined. This pendulant positioning of the patient's breast 28 is advantageous in that the breast 28 is drawn downwardly by gravity, thereby facilitating access to areas of interest within the patient's breast 28 near the chest wall.

The receiver 14 and basket assembly 20 for immobilizing the patient's breast 28 also travel with table top 18 so that a fixed spatial relationship is maintained. As noted above, the receiver 14 is disposed in close proximity to the breast 28 under examination and surrounds the breast 28 so as to provide high resolution, breast specific signal reception. In this regard, the illustrated receiver 14 comprises an RF receiver coil provided in a generally conical configuration. The receiver 14, together with the basket assembly 20, as will be described below, is connected to table top 18 so that the receiver surrounds opening 52 in table top 18 to receive the patient's breast 28. In addition, the receiver 14 communicates with a conventional MRI signal processing unit, which can comprise a digital computer, for acquiring data and reconstructing images based on the received signals. The receiver coils can also be utilized to transmit the RF signal which cooperates with the magnetic field to provide the MRI signal.

Basket assembly 20 is interposed between receiver 14 and the patient's breast 28. The basket assembly 20 immobilizes the patient's breast 28 relative to table top 18 so that localization information derived in the MRI procedure can be used in guiding the medical instrument 26 to an area of interest within the patient's breast 28. Accordingly, the basket assembly 20 is connected to table top 18 to receive the breast 28 protruding through opening 52.

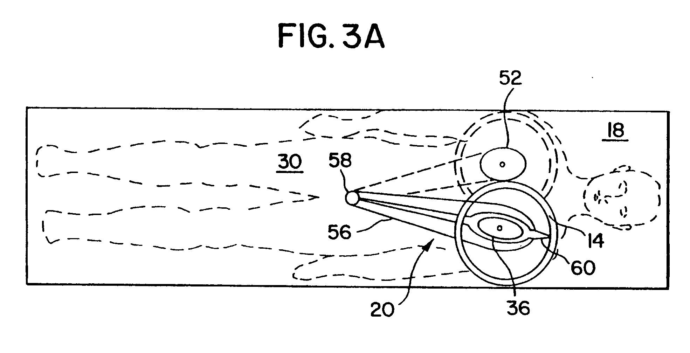

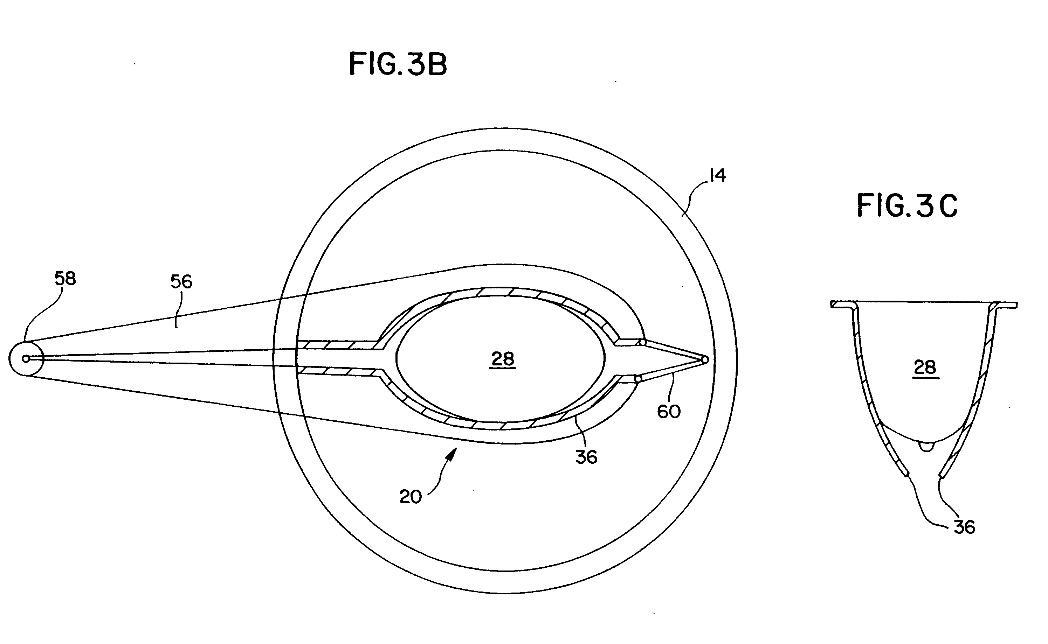

As shown most clearly in Figure 3a, the basket assembly 20 comprises a basket 36 formed in two halves which are drawn together to engage the patient's breast 28. The basket 36 is shaped to generally conform to the contours of the patient's breast 28 thereby enhancing patient comfort. It will be appreciated that it is unnecessary in MRI to compress the breast 28 to a uniform thickness. In addition, the basket 36 includes a number of openings 54 of any suitable shape to allow insertion of the medical instrument 26 therethrough. Additional baskets 36 of various sizes can be provided for interconnection to table top 18 to accommodate patients of different sizes. The basket 36 is formed from a material such as one of various plastics which does not unduly interfere with the MRI procedure.

The two halves of basket 36 are connected via arms 56 to a common non-magnetic hinge 58 and are drawn together by a non-magnetic spring loaded connection 60. Receiver 14 is also mounted to table top 18 via hinge 58 so as to move with basket 36. The hinge 58 is positioned such that the basket 36 and receiver 14 can be moved across the patient's chest from one breast to the other. In this manner, table top 18 can be utilized to perform medical procedures on either breast without awkwardly positioning the patient 30 on table top 18.

The medical instrument insertion assembly 22 is used to insert a medical instrument 26 to an area of interest within the patient's breast 28 identified through MRI. The nature of the medical instrument 26 and insertion assembly 22 depends on the particular procedure to be performed. One such procedure is a needle biopsy to obtain a cell or tissue sample from a suspicious lesion for diagnosing the nature of the lesion, e.g., cancerous, benign, or more specifically, fibroadenoma, ductile carcinoma, etc. Other procedures which can be conducted using the system 10 include various trans-cannula procedures such as cryoablation, laser ablation, hyperthermia, medicament delivery, percutaneous removal, draining or other procedures for treating or eliminating a lesion, cyst or other area of interest. Thus, the insertion assembly 22 can simply be a guide for facilitating manual insertion of a biopsy needle, trocar and cannula, or other instrument, or the assembly can be a mechanized delivery system. In the illustrated embodiment, insertion assembly 22 comprises a biopsy gun for rapid delivery of a biopsy needle to an identified lesion.

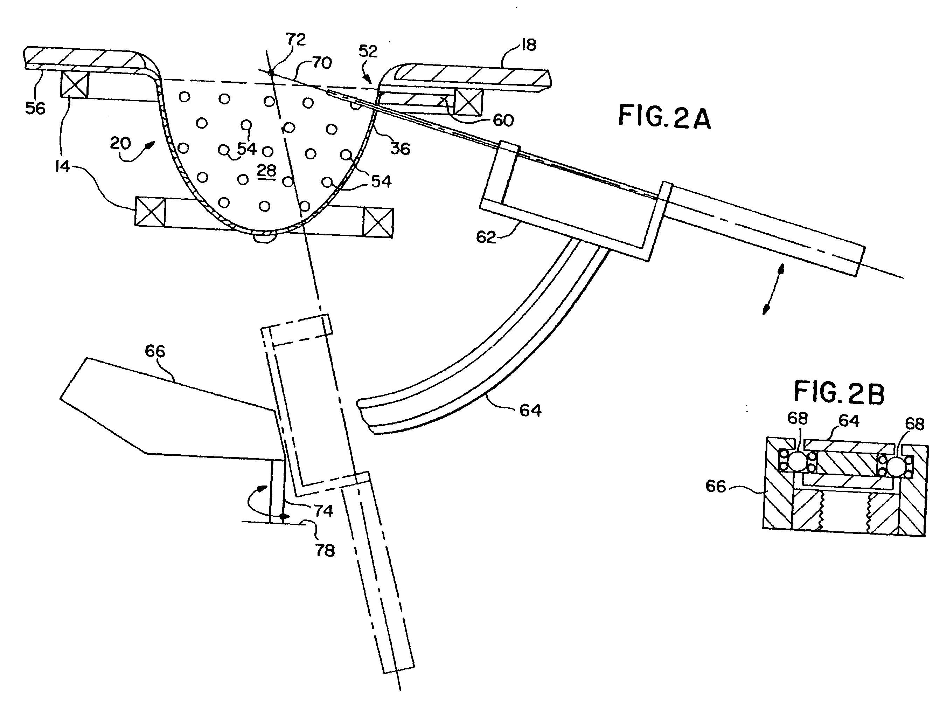

The guidance assembly 24 is used for aiming the medical instrument 26 at the identified area of interest within the patient's breast 28 and allows for selection of a penetration path wherein the receiver 14 and basket 36 are avoided. Accordingly, the guidance assembly 24 allows the medical instrument 26 to pass between the coils of receiver 14 and through an opening 54 of basket 36 en route to the area of interest. The guidance assembly 24 comprises a platform 62 for supporting the medical instrument insertion assembly 22. The platform 62 is mounted on an arcuate rail 64 which is slidably received within housing 66 via bearings 68 (Fig. 2b). In this manner, the insertion assembly 22 can be orbitally moved by sliding rail 64 within housing 66, as shown in phantom in Fig. 2a, such that the aiming axis 70 of medical instrument 26 remains directed at isocenter 72.

In addition, the insertion assembly 22 can be rotationally moved around the patient's breast 28 while the aiming axis 70 of medical instrument 26 remains directed at isocenter 72. Housing 66 is thus mounted on shaft 74 for rotation about shaft axis 76. The intersection of the aiming axis 70 and shaft axis 76 thus defines the isocenter 72. It will thus be appreciated that the combination of orbital and rotational movement of the medical instrument 26 allows for variation of the penetration path of the medical instrument 26 with respect to breast approach angle and with respect to inclination angle relative to the chest wall.

Targeting of an area of interest within the patient's breast is accomplished by positioning the patient 30 and/or the guidance assembly 24 so that the area of interest coincides with the isocenter 72 of guidance assembly 24. In the illustrated embodiment, guidance assembly 24 is moveable relative to stretcher assembly 16 and patient 30. To adjust the height of guidance assembly 24, shaft 74 can be raised or lowered within cylinder 78 mounted on base 80. Base 80 is slidably moveable on recessed surface 78 of platform 38 so that guidance assembly 24 can be moved side-to-side or forward-to-rearward relative to the patient's chest. For example, cylinder 78 can be mounted for forward-to-rearward movement relative to base 80, and base 80 can be mounted for side-to-side movement relative to surface 82. Appropriate brakes may be provided between all moveable components of guidance assembly 24 to lock the assembly into a selected position.

In order to allow for accurate targeting of an area of interest within the patient's breast 28, the spatial relationship between the area of interest and guidance assembly 24 must be known. This can be accomplished by providing at least one reference marker which appears in the images used for localizing the area of interest and which has a known location relative to the guidance assembly 24 when the system 10 is configured for medical device insertion. In the illustrated embodiment, this purpose is served by incorporating three high contrast markers into, or attaching three high contrast markers to the basket 36. The markers may comprise oil capsules and/or may include gadolinium or other contrast materials.

Any of the above-described movements of system can be motorized if desired. However, care must be taken to ensure that such motors do not interfere with and are otherwise compatible with the magnetic field generator 12. In this regard, hydraulic motors may be disposed in a separate, shielded room where the necessary power is transmitted to the appropriate components of system 10 by hydraulic lines.

In summary, the system 10 operates as follows. The stretcher assembly is first positioned adjacent the magnetic field generator 12 and the height of the table top 18 is adjusted relative to the bore 34 of generator 12.

The table top 18 is then extended so that the patient 30 is centrally disposed within bore 34. Receiver 14 is utilized in combination with generator 12 to transmit MRI signals and is also used to receive signals from the tissue under examination. MRI images of the patient's breast 28 are thus obtained. Based on these images, the location of an area of interest within the patient's breast 28 is determined. With the table top 18 in the retracted position, the guidance assembly 24 is positioned so that the isocenter 72 thereof coincides with the identified area of interest. The guidance assembly can then be manipulated to select a penetration path for medical instrument 26 wherein interference with the receiver 24 and basket 36 is avoided.

While various embodiments of the present invention have been described in detail, it is apparent that further modifications and adaptations of the invention will occur to those skilled in the art. However, it is to be expressly understood that such modifications and adaptations are only limited by the scope of the appended claims.

## Claims

1. An apparatus (10) for use in performing medical procedures on a patients breast (28), comprising:
immobilizing means (36) for immobilizing said patient's breast (28);
magnetic resonance imaging means for imaging said patient's breast (28) including transmitting means (12) for applying a signal to said patient's breast (28) and receiving means (14) for receiving a signal from said patient's breast (28), said receiving means (14) defining a volume dimensioned for receiving said patient's breast (28), wherein said magnetic resonance imaging means provides imaging information regarding said patient's breast (28);
identifying means employing said imaging information for identifying the location of an area of interest within said patient's breast (28); and
medical instrument means (22) for inserting a medical instrument (26) to said identified location said area of interest within said patient's breast (28); **characterized by**
isocentric support means for movably supporting said medical instrument (26) so that said medical instrument (26) is moveable between a first position and a second position wherein said medical instrument (26) is aimed at an isocenter of said support means in each of said first and second positions,
table means (18) for supporting said patient in a prone position, said table means having an opening (56) therein through which said patient's breast (28) is permitted to pendulantly protrude,
positioning means for moving said table means (18) between a first position wherein said patient's breast (28) is positioned for imaging and a second position wherein said patient's breast (28) is positioned for insertion of said medical instrument (26),
wherein said patient is movable, together with said immobilizing means (36) and said receiving means (14) relative to said isocentric support means between a first position where said patient's breast (28) is positioned for imaging using said magnetic resonance imaging means and a second position where said patient's breast (28) is positioned for insertion of said medical instrument using said isocentric support means.

2. The apparatus of Claim 1, wherein said immobilizing means (36) comprises a contractible basket shaped to generally conform to the contours of said patient's breast (28).

3. The apparatus of Claim 1, wherein said immobilizing means (36) comprises a basket formed two halves which are urged together by a resilient member.

4. The apparatus of Claim 1, wherein said magnetic resonance imaging means comprises cylindrical magnetic means for receiving said patient therein.

5. The apparatus of Claim 1, further comprising penetration path selection means for selecting a penetration path for inserting said medical instrument (26) to said identified location of said area of interest within said patient's breast (28) wherein the location of at least one of said immobilizing means (36) and said receiving means (12) is accounted for in selecting said penetration path.

6. The apparatus of Claim 1, further comprising reference mark means for providing reference marks useful for establishing a frame of reference for analyzing images of said patient's breast (28).

7. The apparatus of Claim 6, wherein said reference mark means comprises a contrast material disposed in predetermined spatial location relative to said immobilization means.

8. The apparatus of Claim 1, wherein said medical instrument means (22) comprises a biopsy gun for rapid insertion of a biopsy needle to said identified location of said area of interest within said patient's breast (28).

9. The apparatus of Claim 1, wherein said support means is operative for orbitally moving said medical instrument (26) across a range of positions relative to said isocenter, said range of positions defining a substantially planar region.

10. The apparatus of Claim 1, wherein said support means is operative for rotationally moving said medical instrument (26) across a range of positions relative to said isocenter, said range of positions defining a three dimensional region.

11. The apparatus of Claim 1, wherein said magnetic resonance imaging means includes a generally cylindrical component (32) defining an internal axial bore (34; and
said positioning means are for axially moving said table means relative to said bore (34) between the first position wherein said patient is disposed within said bore for imaging said patient's breast (28) and the second position wherein said patient is disposed outside said bore, wherein said patient's breast (28) is maintained in said same fixed position with respect to said predetermined frame of reference when said table means is in said first position and when said table means is in said second position.

12. The apparatus of Claim 11, wherein said immobilizing means (36) is interconnected to said table means so as to allow for movement of said receiving means (12) across said patient's chest.

## Patentansprüche

1. Vorrichtung (10) für den Einsatz in der Durchführung von medizinischen Verfahren an einer Patientenbrust (28), umfassend:
Festlegungsvorrichtung (36) für die Immobilisierung der Patientenbrust (28);
Magnetresonanzbildgebungsvorrichtung für die Abbildung der Patientenbrust (28) umfassend eine Sendevorrichtung (12) für das Anwenden eines Signals auf die Patientenbrust (28) und Empfangsvorrichtung (14) für den Empfang eines Signals von der Patientenbrust (28), wobei die Empfangsvorrichtung (14) ein Volumen definiert, das für den Empfang der Patientenbrust (28) dimensioniert ist, worin die Magnetresonanz-Bildgebungsvorrichtung Abbildungsinformationen bezüglich der Patientenbrust (28) liefert;
Erkennungsvorrichtung, die die Abbildungsinformation für die Lageerkennung eines interessierenden Bereichs innerhalb der Patientenbrust (28) nutzt;
und eine medizinische Instrumentenvorrichtung (22) für das Einführen eines medizinischen Instruments (26) in die erkannte Stelle des interessierenden Bereichs innerhalb der Patientenbrust (28); **gekennzeichnet durch**
eine isozentrische Unterstützungsvorrichtung für bewegliche Unterstützung des medizinischen Instruments (26), so dass das medizinische Instrument (26) zwischen einer ersten Position und einer zweiten Position beweglich ist, worin das medizinische Instrument (26) auf das Isozentrum der Unterstützungsvorrichtung in jeder der ersten und zweiten Positionen gerichtet ist,
Tischvorrichtung (18) für das Unterstützen des Patienten in einer Bauchlage, wobei die Tischvorrichtung eine Öffnung (56) hat, **durch** welche es der Patientenbrust (28) ermöglicht ist pendelnd herauszuragen, Positionierungsvorrichtung für das Bewegen der Tischvorrichtung (18) zwischen einer ersten Position, worin die Patientenbrust (28) für die Abbildung positioniert ist und einer zweiten Position, worin die Patientenbrust (28) für die Einführung des medizinischen Instruments (26) positioniert ist,
worin der Patient bewegbar ist, zusammen mit der Immobilisierungsvorrichtung (36) und der Empfangsvorrichtung (14) relativ zu der isozentrischen Unterstützungsvorrichtung zwischen einer ersten Position, wo die Patientenbrust (28) für die Abbildung mittels der Magnetresonanzbildgebungsvorrichtung positioniert ist und einer zweiten Position, wo die Patientenbrust (28) positioniert ist, für die Einführung des medizinischen Instruments mittels der isozentrischen Unterstützungsvorrichtung.

2. Vorrichtung gemäß Anspruch 1, **dadurch gekennzeichnet, dass** die Immobilisierungsvorrichtung (36) einen zusammenziehbaren Korb umfasst, so geformt, dass er sich den Konturen der Patientenbrust (28) generell anpasst.

3. Vorrichtung gemäß Anspruch 1, **dadurch gekennzeichnet, dass** die Immobilisierungsvorrichtung (36) einen Korb umfasst, gebildet aus zwei Hälften, welche durch ein federndes Stabelement zusammen gedrängt sind.

4. Vorrichtung gemäß Anspruch 1, **dadurch gekennzeichnet, dass** die Magnetresonanz-Bildgebungsvorrichtung eine zylindrische magnetische Vorrichtung umfasst um den Patienten darin aufzunehmen.

5. Vorrichtung gemäß Anspruch 1, **dadurch gekennzeichnet, dass** sie eine Durchstosswegauswahl-Vorrichtung für die Auswahl eines Durchstosswegs für das Eindringen des medizinischen Instruments (26) in die erkannte Lage des interessierenden Bereichs innerhalb der Patientenbrust (28) umfasst, worin die Lage mindestens einer Immobilisierungsvorrichtung (36) und der Empfangsvorrichtung (12) für die Auswahl des Durchstosswegs dafür ausgewiesen ist.

6. Vorrichtung gemäß Anspruch 1, **dadurch gekennzeichnet, dass** sie eine Referenzmarkierungsvorrichtung umfasst für das Vorsehen von Referenzmarkierungen, dienlich für das Festlegen eines Referenzrahmens für die Bildanalyse der Patientenbrust (28).

7. Vorrichtung gemäß Anspruch 6, **dadurch gekennzeichnet, dass** die Referenzmarkierungsvorrichtung ein Kontrastmaterial umfasst, bereitgestellt in einer vorbestimmten räumlichen Lage, relativ zu der Immobilisierungsvorrichtung.

8. Vorrichtung gemäß Anspruch 1, **dadurch gekennzeichnet, dass** die medizinische Instrumentenvorrichtung (22) eine Biopsiekanone umfasst, für das schnelle Einführen einer Biopsienadel in eine erkannte Lage des interessierenden Bereichs innerhalb der Patientenbrust (28).

9. Vorrichtung gemäß Anspruch 1, **dadurch gekennzeichnet, dass** die Unterstützungsvorrichtung wirksam ist für eine kreisförmige Bewegung des medizinischen Instruments (26) über einen Positionsbereich relativ zu dem Isozentrum, wobei der Positionsbereich eine im wesentlichen flache Region definiert.

10. Vorrichtung gemäß Anspruch 1, **dadurch gekennzeichnet, dass** die Unterstützungsvorrichtung wirksam ist für die Drehbewegung des medizinischen Instruments (26) über einen Positionsbereich relativ zu dem Isozentrum, wobei der Positionsbereich eine dreidimensionale Region definiert.

11. Vorrichtung gemäß Anspruch 1, **dadurch gekennzeichnet, dass** die Magnetresonanz-Bildgebungsvorrichtung im allgemeinen eine zylindrische Komponente (32) umfasst, die eine interne Axialbohrung (34) definiert;
und die Positiononierungsvorrichtung vorgesehen ist für die axiale Bewegung der Tischvorrichtung relativ zu der Bohrung (34), zwischen der ersten Position, worin der Patient innerhalb der Bohrung bereit ist für die Abbildung der Patientenbrust (28) und der zweiten Position, worin der Patient außerhalb der Bohrung (34) bereit ist, worin die Patientenbrust (28) in der selben fixierten Position beibehalten wird bezüglich des vorbestimmten Referenzrahmens, wenn die Tischvorrichtung in der ersten Position ist und wenn die Tischvorrichtung in der zweiten Position ist.

12. Vorrichtung gemäß Anspruch 11, **dadurch gekennzeichnet, dass** die Immobilisierungsvorrichtung (36) mit der Tischvorrichtung verbunden ist, um die Bewegung der Empfangsvorrichtung (12) über den Patientenbrustkorb zu bewegen.

## Revendications

1. Appareil (10) adapté pour exécuter des interventions médicales sur le sein d'un patient (28), comprenant :
des moyens d'immobilisation (36) adaptés pour immobiliser ledit sein du patient (28) ;
des moyens d'imagerie par résonance magnétique adaptés pour prendre des images dudit sein du patient (28) comprenant des moyens de transmission (12) adaptés pour appliquer un signal sur ledit sein du patient (28) et des moyens de réception (14) adaptés pour recevoir un signal à partir dudit sein du patient (28), lesdits moyens de réception (14) définissant un volume dimensionné pour recevoir ledit sein du patient dans lequel lesdits moyens d'imagerie par résonance magnétique procurent des informations de formation d'images relatives audit sein du patient (28) ;
des moyens d'identification employant lesdites informations de formation d'images afin d'identifier l'emplacement d'une zone d'intérêt à l'intérieur dudit sein du patient (28) ; et
des moyens d'instrumentation médicale (22) adaptés pour introduire un instrument médical (26) au niveau dudit emplacement identifié de ladite zone d'intérêt à l'intérieur dudit sein du patient (28) ; **caractérisé par**
des moyens de support isocentrique adaptés pour supporter ledit instrument médical (26) de façon amovible de sorte que ledit instrument médical (26) est susceptible de se déplacer entre une première position et une deuxième position dans lesquelles ledit instrument médical (26) est dirigé vers un isocentre desdits moyens de support dans chacune desdites première et deuxième positions ;
des moyens formant table (18) adaptés pour soutenir ledit patient dans une position de décubitus ventral, lesdits moyens formant table ayant une ouverture (56) dans ceux-ci à travers laquelle ledit sein du patient (28) est autorisé à saillir de façon retombante ;
des moyens de positionnement adaptés pour déplacer lesdits moyens formant table (18) entre une première position dans laquelle ledit sein du patient (28) est positionné en vue de la prise d'images et une deuxième position dans laquelle ledit sein du patient (28) est positionné en vue de l'introduction dudit instrument médical (26) ;
dans lequel ledit patient est susceptible de se déplacer, conjointement avec lesdits moyens d'immobilisation (36) et lesdits moyens de réception (14) par rapport auxdits moyens de support isocentrique entre une première position dans laquelle ledit sein du patient (28) est positionné en vue de la prise d'images à l'aide desdits moyens d'imagerie par résonance magnétique et une deuxième position dans laquelle ledit sein du patient (28) est positionné en vue de l'introduction dudit instrument médical à l'aide desdits moyens de support isocentrique.

2. Appareil selon la revendication 1, dans lequel lesdits moyens d'immobilisation (36) comprennent un panier susceptible de se contracter, d'une forme qui épouse généralement les contours dudit sein du patient (28).

3. Appareil selon la revendication 1, dans lequel lesdits moyens d'immobilisation (36) comprennent un panier composé de deux moitiés qui sont pressées l'une contre l'autre par un organe résilient.

4. Appareil selon la revendication 1, dans lequel lesdits moyens d'imagerie par résonance magnétique comprennent des moyens magnétiques cylindriques adaptés pour recevoir ledit patient à l'intérieur de ceux-ci.

5. Appareil selon la revendication 1, comprenant en outre des moyens de sélection d'une trajectoire de pénétration adaptés pour sélectionner une trajectoire de pénétration en vue de l'introduction dudit instrument médical (26) au niveau dudit emplacement identifié de ladite zone d'intérêt à l'intérieur dudit sein du patient (28) dans lesquels l'emplacement d'au moins un desdits moyens d'immobilisation (36) et desdits des moyens de réception (12) est pris en considération pour la sélection de ladite trajectoire de pénétration.

6. Appareil selon la revendication 1, comprenant en outre des moyens de marquage de référence adaptés pour fournir des marques de référence utiles pour établir un cadre de référence en vue de l'analyse des images dudit sein du patient (28).

7. Appareil selon la revendication 6, dans lequel lesdits moyens de marquage de référence comprennent un produit de contraste disposé au niveau d'un emplacement spatial prédéterminé par rapport auxdits moyens d'immobilisation.

8. Appareil selon la revendication 1, dans lequel lesdits moyens d'instrumentation médicale (22) comprennent un pistolet à biopsie adapté pour permettre l'introduction rapide d'une aiguille à biopsie au niveau dudit emplacement identifié de ladite zone d'intérêt à l'intérieur dudit sein du patient (28).

9. Appareil selon la revendication 1, dans lequel lesdits moyens de support sont opérationnels pour déplacer ledit instrument médical (26) de façon orbitale sur une plage de positions par rapport audit isocentre, ladite plage de positions définissant une région sensiblement plane.

10. Appareil selon la revendication 1, dans lequel lesdits moyens de support sont opérationnels pour déplacer ledit instrument médical (26) de façon rotative sur une plage de positions par rapport audit isocentre, ladite plage de positions définissant une région à trois dimensions.

11. Appareil selon la revendication 1, dans lequel lesdits moyens d'imagerie par résonance magnétique comprennent un composant généralement cylindrique (32) définissant un orifice axial interne (34) ; et dans lequel lesdits moyens de positionnement sont adaptés pour déplacer lesdits moyens formant table axialement par rapport audit orifice (34) entre la première position dans laquelle ledit patient se trouve placé à l'intérieur dudit orifice en vue de la prise d'images dudit sein du patient (28) et la deuxième position dans laquelle ledit patient se trouve placé à l'extérieur dudit orifice, dans lequel ledit sein du patient (28) est maintenu dans la même position fixe par rapport audit cadre de référence prédéterminé lorsque lesdits moyens formant table se trouvent dans ladite première position et lorsque lesdits moyens formant table se trouvent dans ladite deuxième position.

12. Appareil selon la revendication 11, dans lequel lesdits moyens d'immobilisation (36) sont raccordés auxdits moyens formant table de manière à permettre auxdits moyens de réception (12) de se déplacer sur toute l'étendue dudit sein du patient.
